# EUROPÄISCHE PATENTSCHRIFT

(11) **EP 1 790 306 B1**
(45) Veröffentlichungstag und Bekanntmachung des Hinweises auf die Patenterteilung: **11.06.2008**
(21) Anmeldenummer: 05025655.1
(22) Anmeldetag: 24.11.2005
(51) Int. Cl.: A61B 19/00

(54) **Ansteuerung einer medizintechnischen Navigationssoftware mit Klicksignal**
Control of a medical navigation software through a click signal
Control d'un logiciel de navigation médicale avec l'aide d'un signal clic

(43) Veröffentlichungstag der Anmeldung: 30.05.2007
(73) Patentinhaber: BrainLAB AG, 85551 Kirchheim/Heimstetten (DE)
(72) Erfinder: Immerz, Martin, 82166 Gräfelfing (DE); Steinle, Wolfgang, 80337 München (DE)
(74) Vertreter: Schwabe - Sandmair - Marx

(56) Entgegenhaltungen:
- EP-A- 0 959 401
- EP-A- 1 523 950
- WO-A-01/47413
- WO-A-03/086190
- US-A1- 2004 013 240
- US-A1- 2004 074 652

## Beschreibung

Die Erfindung betrifft die Ansteuerung einer medizintechnischen Navigationssoftware. Insbesondere betrifft sie ein Verfahren zur Bereitstellung eines geeigneten Teils einer medizinischen Navigation sowie eine Vorrichtung, die zu diesem Zweck verwendet werden kann.

In der computerunterstützten Chirurgie werden dem Behandelnden - meist von einem Navigations- bzw. Trackingsystem im Operationssaal - unterschiedliche Bildausgaben zur Verfügung gestellt. Diese Bildausgaben zeigen Patientendatensätze oder zumindest Teile davon, also z.B. dreidimensionale oder Schnitt-Wiedergaben von Patientenkörperteilen. Die Patientendatensätze können entweder durch bildgebende Verfahren wie z.B. CT- oder MR-Tomographie, Röntgen, Ultraschall oder Fluoroskopie, oder durch bildlose Verfahren wie das Abgreifen der Knochenoberfläche mittels eines registrierten Zeigeinstruments oder Laserabtastung erzeugt werde. Ferner können im Rahmen einer Bildunterstützung beispielsweise Instrumente oder Behandlungseinrichtungen im Positionsverhältnis zu den Patientendaten gezeigt werden, um dem Behandelnden so eine Unterstützung zu gewährleisten.

Je nach Behandlungsfortschritt wird es oft notwendig, einen speziellen Teil der Softwareunterstützung auf der Bildschirmausgabe anzuzeigen, nämlich den Teil mit den Funktionen, die für den momentanen Behandlungsschritt gerade erforderlich sind. Man könnte auch sagen, dass die Software aus verschiedenen "Seiten" besteht, die im Laufe der Behandlung durchwechseln.

Bei bildunterstützten Anwendungen im Bereich der Hüfte oder des Knies ist es ebenbeispielsweise öfter notwendig, während der Behandlung bestimmte Software-Seiten anzuwählen. Dies wird derzeit hauptsächlich mittels eines berührungsempfindlichen Bildschirms (touch screen) bewerkstelligt, auf dem per Druck die entsprechenden Eingaben gemacht werden. Auch andere Arten der Software-Ansteuerung sind bekannt, und sie umfassen beispielsweise die Verwendung eines Fußschalters, einer virtuellen Tastatur, einer Sprachsteuerung oder navigierter bzw. betätigbarer Instrumente, die eine bestimmte Tätigkeit ausführen, welche einen Einfluss auf die Softwaresteuerung hat.

Oftmals wird es von den Behandelnden als sehr störend empfunden, dass sie sich neben ihrer Operationstätigkeit noch zusätzlich dem "Umschalten" der Software widmen müssen. Darunter leidet die Akzeptanz der ansonsten als hoch vorteilhaft angesehenen Bildunterstützung.

Auch die akustische Ansteuerung mit gesprochenen Befehlen hat sich als nicht befriedigend herausgestellt. Dokument WO 03/086190 offenbart eine solche Ansteuerung. So genannte "voice-controlled" Ansteuerungssysteme treffen bei den Behandelnden oftmals auf Widerstände, da es vielen Menschen einfach nicht leicht fällt, mit einem Computer zu sprechen, speziell wenn andere dabei sind. Außerdem wird während einer Behandlung im Team normalerweise gesprochen, und es besteht die Gefahr, dass das Steuerungssystem dadurch in die Irre geführt wird. Ferner tragen die Behandelnden in fast allen Fällen Gesichtsmasken oder Spritzschutz bzw. andere Gesichtsschutzeinrichtungen. Gesprochene akustische Signale können dadurch verzerrt und für das System unkenntlich werden.

Es ist die Aufgabe der vorliegenden Erfindung, die Ansteuerung eines Navigationssystems bzw. einer chirurgischen Bildunterstützung zu verbessern. Insbesondere soll auch sichergestellt werden, dass dem Behandelnden immer zuverlässig eine gerade benötigte Software oder "Software-Seite" zur Verfügung steht.

Diese Aufgabe wird erfindungsgemäß durch ein Verfahren gemäß dem Anspruch 1 und eine Vorrichtung gemäß dem Anspruch 8 gelöst. Die Unteransprüche beschreiben bevorzugte Ausführungsformen der Erfindung.

Die vorliegende Erfindung betrifft ganz allgemein ein Verfahren zur Ansteuerung eines computerunterstützten, medizinischen Navigationssystems, durch das eine Unterstützung für einen bestimmten Teil einer Behandlung gewährleistet wird, wobei ein Klicksignal erzeugt und vom Navigationssystem empfangen wird, worauf das Navigationssystem die Behandlungsunterstützung bewirkt.

Mit anderen Worten wird erfindungsgemäß zum Auslösen einer bestimmten Aktion (z.B. in der Bildausgabe) ein akustisches Signal erzeugt, das einwandfrei unterscheidbar und identifizierbar ist. Klicksignale sind einfach und kurz, und sie können leicht in einer Lautstärke bereitgestellt werden, die oberhalb der "Zimmerlautstärke", das heißt der normalen Lautstärke während einer medizinischen Behandlung liegt. Ein Klicksignal ist ausgeprägt, deutlich, eindeutig, vernehmlich und unterscheidbar von anderen akustischen Signalen in einem Operationsraum. Insbesondere ist es leicht zur Verfügung zu stellen, nämlich indem ein Gegenstand bereitgestellt wird, der eine sprunghafte Formänderung aufgrund einer Manipulation durchführt. Es kann aber auch grundsätzlich durch einen Stoß bzw. das Auftreffen eines Körpers auf einen anderen erzeugt werden.

Die erfindungsgemäß verwirklichte Ansteuerung ist sehr stabil, insbesondere im Vergleich mit einer Sprachansteuerung. Das eindeutig unterscheidbare akustische Klicksignal kann leicht erfasst und ausgefiltert werden. Gegenüber einem solchen Gerät dürften Behandelnde auch keinerlei Widerwillen zeigen (im Vergleich zu dem "Reden mit einem Computer"). Ein Klicksignal kann durch andere Geräusche kaum gestört werden, und es hat eine einzigartige Frequenz, wobei es sehr stabil ausgebildet werden kann, verlässlich ist und auch leicht zu reinigen oder zu autoklavieren oder sterilisieren ist.

Erfindungsgemäß verwendete Klickmechanismen benötigen keine Batterien oder Kabel, und sie sind leicht und billig herzustellen.

Die vorliegende Erfindung umfasst gemäß einer Ausführungsform ein Verfahren zur Bereitstellung eines geeigneten Teils eines computerunterstützten, medizintechnischen Navigationssystems, wobei im Rahmen der Navigation unterschiedliche Bildausgaben erfolgen, die eine Unterstützung für einen Teil einer Behandlung gewährleisten. Es wird ein Klicksignal erzeugt und vom Navigationssystem empfangen, worauf das Navigationssystem das Wechseln der Bildausgabe bewirkt. Mit anderen Worten betrifft die Erfindung in einer bevorzugten Ausführungsform ein Verfahren, bei dem im Rahmen der Navigation unterschiedliche Bildausgaben erfolgen, und bei dem der Empfang des Klicksignals das Wechseln der Bildausgabe bewirkt.

Das Klicksignal kann das Um- oder Weiterschalten oder das Zurückschalten der Bildausgabe auslösen, und es kann einerseits mit einem separaten Klickmechanismus erzeugt werden oder andererseits mit einem Klickmechanismus erzeugt werden, der an einem medizinischen Instrument angeordnet ist.

Es besteht im Rahmen der Erfindung die Möglichkeit, zwei oder mehrere im Ton, insbesondere in der Tonhöhe verschiedene Klicksignale zu erzeugen. Zusätzlich oder in Kombination hiermit können in unterschiedlichem Rhythmus aufeinander folgende Klicksignale erzeugt werden, also beispielsweise zwei schnell aufeinander folgende Klicks oder zwei langsam aufeinander folgende Klicks. Dadurch werden verschiedene und vielseitige Optionen bereitgestellt, um unterschiedliche Aktionen zu bewirken, insbesondere zum Weiterschalten oder Zurückschalten der Bildausgabe in einer Ausgabefolge.

Die erfindungsgemäße Vorrichtung zur Ansteuerung eines medizintechnischen Navigationssystems umfasst ein solches medizinischen Navigations- und/oder Bildunterstützungssystem und eine Steuerung für das System in Reaktion auf eine Eingabe, wobei die Steuerung eine Klickvorrichtung und eine dem Navigationssystem zugeordnete Empfangsvorrichtung für ein Klicksignal umfasst.

Die Steuerung kann im Navigationssystem integriert sein und eine Schalteinheit zum Um- oder Weiterschalten oder Zurückschalten der Bildausgabe auf das Klicksignal hin umfassen. Die Vorrichtung kann einen separaten Klickmechanismus umfassen oder einen Klickmechanismus, der an einem medizinischen Instrument angeordnet ist, insbesondere abnehmbar und/oder austauschbar angeordnet ist. Bei einer Ausführungsform der Erfindung umfasst der Klickmechanismus ein verformbares Flächengebilde, vorzugsweise aus Metall. Dieses Flächengebilde kann durch eine mechanische Verformung eine sprunghafte Umformung erfahren und dabei das Klicksignal von sich geben. Ganz allgemein besteht eine mögliche Art für die Funktion des Klickmechanismus darin, dass eine sprunghafte Formänderung eines Körpers die Erzeugung des Signals hervorruft. Gute Klicksignale erhält man beispielsweise, wenn ein vorgeformter Abschnitt sprunghaft verformt wird, beispielsweise eine Delle oder Ausbuchtung in einem Metallstreifen. Ein gut unterscheidbares Klickgeräusch entsteht insbesondere dann, wenn man diesen Metallkörper derart manipuliert, dass die Delle sich zur anderen Flächenseite hin umkehrt (und eventuell auch wieder in die Ausgangslage zurückkehrt).

Der Klickmechanismus kann gemäß einer Ausführungsvariante zwei oder mehrere im Ton, insbesondere in der Tonhöhe verschiedene Klicksignale erzeugen, und zwar speziell durch Umformung verschiedener Abschnitte oder des gleichen Abschnittes in unterschiedlichen Richtungen.

Natürlich besteht im Rahmen der vorliegenden Erfindung auch die Möglichkeit, zwei oder mehrere Klickmechanismen zur Erzeugung unterschiedlicher Klicksignale bereitzustellen.

Gemäß einer Ausführungsform der Erfindung umfasst das Navigationssystem eine Bildausgabeeinheit, und die Steuerung steuert die Darstellung auf der Bildausgabeeinheit. Speziell wird dabei ein Bildausgabewechsel bewirkt, insbesondere zum Weiterschalten oder Zurückschalten der Bildausgabe in einer Bildausgabefolge eines Software-Workflows.

Anders ausgedrückt dient die erfindungsgemäße Vorrichtung gemäß einer Ausführungsvariante der Bereitstellung eines geeigneten Teils eines medizinischen Navigationssystems bzw. einer geeigneten Bildausgabe eines medizinischen Navigationssystems, und sie weist folgende Elemente auf: ein medizintechnisches Navigations- und/oder Bildunterstützungssystem, das eine Bildausgabeeinheit umfasst, und eine Steuerung für die Darstellung auf der Bildausgabeeinheit in Reaktion auf eine Eingabe.

Die Vorrichtung und ihre Ausführungsformen stellen die oben schon beschriebenen Vorteile zur Verfügung.

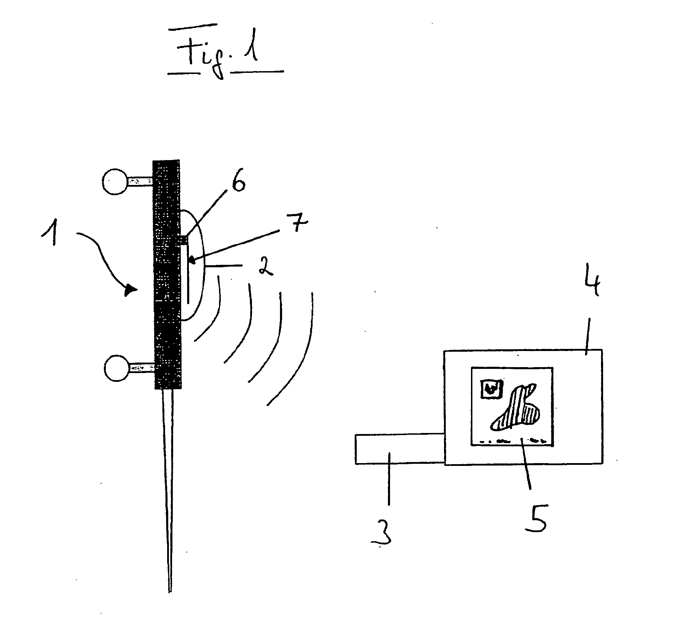

Die Erfindung wird im Weiteren anhand einer Ausführungsform näher erläutert. Sie kann alle hierin beschriebenen Merkmale einzeln oder in jedweder Kombination umfassen. Die beiliegende einzige Figur 1 zeigt eine erfindungsgemäße Vorrichtung zur Ansteuerung eines medizintechnischen Navigationssystems mit einem Klicksignal.

In der Zeichnung der Figur 1 zeigt das Bezugszeichen 1 auf ein chirurgisches Instrument, hier auf eine Sonde mit nicht bezeichneten Navigations-Reflektorenmarkern. Am Griff der Sonde ist ein Klickmechanismus 2 schematisch angedeutet, und dieser Klickmechanismus kann ebenso schematisch in eine Halterung 6 und ein Metallplättchen 7 unterteilt werden. Am medizinischen Instrument 1 angeordnet, ist der Klickmechanismus 2 für den Behandelnden immer erreichbar. Natürlich muss der Mechanismus nicht wie in der Figur dargestellt, seitlich am Griff angeordnet sein, er könnte ebenfalls beispielsweise an der hinteren Stirnseite als eine Art Knopfschalter vorgesehen werden, wobei er dort vorteilhafterweise weniger beim Halten des Instruments stören sollte.

In der dargestellten Ausführungsform weist der Klickmechanismus 2 das Metallplättchen 7 auf, das ein unterscheidbares akustisches Signal abgibt, wenn es deformiert wird, nämlich ein Klicksignal. Wenn dieses unterscheidbare Klickgeräusch durch ein Mikrophon 3 am Navigationssystem 4 erfasst wird, können vordefinierte Softwareschritte aktiviert werden. In den meisten Fällen wird die Anwendung eine solche sein, die ein Durchschreiten einer Arbeitsfolge (Workflow) mit unterschiedlichen Software-Bildschirmseiten gestattet. In der Figur 1 ist beispielsweise der Bildschirm 5 dargestellt, der eine Darstellung zeigt, welche momentan die Arbeit des Behandelnden unterstützt. Wenn eine bestimmte Tätigkeit abgeschlossen ist, kann dann durch die Erzeugung eines Klicksignals mit dem Klickmechanismus 2 eine weitere Bildschirmseite aufgerufen werden, welche die nächste Tätigkeit des Behandelnden explizit unterstützt.

Grundsätzlich ist es möglich, jedwede Aktionen über ein solches Klickgeräusch zur Behandlungsunterstützung auszulösen, und andere mögliche Aktionen könnten beispielsweise das Einspielen von Bilddatensätzen in das Navigationssystem, der Beginn einer Registrierung oder Nachregistrierung für Patientenkörperteile oder Instrumente und das Starten bzw. Anfahren eines behandlungsunterstützenden Geräts sein.

Obwohl in der Zeichnung nicht dargestellt, ist es im Rahmen der vorliegenden Erfindung auch denkbar, beispielsweise zwei unterschiedliche Plättchen zu verwenden, die unterschiedliche akustische Signale abgeben, wenn sie verformt werden, so dass verschiedene Funktionalitäten ermöglicht werden, wie zum Beispiel ein "Vorwärtsschalten" oder ein "Rückwärtsschalten" in einer bestimmten Abfolge. Natürlich können solche Ausführungsformen mit der Bereitstellung mehrerer unterschiedlicher Klicksignale auch anders aufgebaut sein, beispielsweise als Wippschalter.

## Patentansprüche

1. Verfahren zur Ansteuerung eines computerunterstützten, medizintechnischen Navigationssystems (4), durch das eine Unterstützung für einen bestimmten Teil einer Behandlung gewährleistet wird, **dadurch gekennzeichnet, dass** ein Klicksignal erzeugt und vom Navigationssystem empfangen wird, worauf das Navigationssystem (4) die Behandlungsunterstützung bewirkt.

2. Verfahren nach Anspruch 1, bei dem das Klicksignal mit einem separaten Klickmechanismus erzeugt wird.

3. Verfahren nach Anspruch 1, bei dem das Klicksignal mit einem Klickmechanismus (2) erzeugt wird, der an einem medizinischen Instrument (1) angeordnet ist.

4. Verfahren nach einem der Ansprüche 1 bis 3, bei dem zwei oder mehrere, insbesondere im Ton verschiedene und/oder in unterschiedlichem Rhythmus aufeinander folgende, Klicksignale erzeugt werden.

5. Verfahren nach einem der Ansprüche 1 bis 4, bei dem im Rahmen der Navigation unterschiedliche Bildausgaben erfolgen, und bei dem der Empfang des Klicksignals das Wechseln der Bildausgabe bewirkt.

6. Verfahren nach Anspruch 5, bei dem das Klicksignal das Um- oder Weiterschalten oder das Zurückschalten der Bildausgabe auslöst, um unterschiedliche Bildausgabewechsel zu bewirken.

7. Verfahren nach einem der Ansprüche 1 bis 4, bei dem eine oder mehrere der folgenden Aktionen durch das Klicksignal ausgelöst werden:
- das Einspielen von Blüddatensätzen in das Navigationssystem;
- der Beginn einer Registrierung oder Nachregistrierung für Patientenkörperteile oder Instrumente; und
- das Starten bzw. Anfahren eines behandlungsunterstützenden Geräts.

8. Vorrichtung zur Ansteuerung eines medizintechnischen Navigationssystems (4), mit einem medizintechnischen Navigations- und/oder Bildunterstützungssystem (4) und mit einer Steuerung für das System in Reaktion auf eine Eingabe, **dadurch gekennzeichnet, dass** die Steuerung eine Klickvorrichtung (2) und eine dem Navigationssystem zugeordnete Empfangsvorrichtung (3) für ein Klicksignal umfasst.

9. Vorrichtung nach Anspruch 8, **dadurch gekennzeichnet, dass** sie einen separaten Klickmechanismus umfasst.

10. Vorrichtung nach Anspruch 8, **dadurch gekennzeichnet, dass** sie einen Klickmechanismus (2) umfasst, der an einem medizinischen Instrument (1) angeordnet ist, insbesondere abnehmbar und/oder austauschbar angeordnet ist.

11. Vorrichtung nach einem der Ansprüche 8 bis 10, **dadurch gekennzeichnet, dass** die Steuerung im Navigationssystem (4) integriert ist.

12. Vorrichtung nach einem der Ansprüche 8 bis 12, **dadurch gekennzeichnet, dass** der Klickmechanismus ein verformbares Flächengebilde, vorzugsweise aus Metall, umfasst, welches durch eine mechanische Verformung eine sprunghafte Umformung eines, insbesondere vorgeformten, Abschnittes erfährt.

13. Vorrichtung nach einem der Ansprüche 8 bis 12, mit einem Klickmechanismus, der zwei oder mehrere im Ton, insbesondere in der Tonhöhe verschiedene Klicksignale erzeugen kann, insbesondere durch Umformung verschiedener Abschnitte oder des gleichen Abschnittes in unterschiedlichen Richtungen.

14. Vorrichtung nach einem der Ansprüche 8 bis 13, **dadurch gekennzeichnet, dass** sie zwei oder mehrere Klickmechanismen zur Erzeugung unterschiedlicher Klicksignale aufweist.

15. Vorrichtung nach einem der Ansprüche 8 bis 14, **dadurch gekennzeichnet, dass** das Navigationssystem eine Bildausgabeeinheit (5) umfasst und die Steuerung die Darstellung auf der Bildausgabeeinheit (5) steuert, speziell um Bildausgabewechsel zu bewirken, insbesondere zum Weiterschalten oder Zurückschalten der Bildausgabe in einer Bildausgabefolge eines Software-Workflows.

## Claims

1. A method for controlling a computer-assisted, medical navigation system (4), which ensures assistance for a particular part of a treatment, **characterised in that** a click signal is generated and received by the navigation system, whereupon the navigation system (4) assists the treatment.

2. The method according to claim 1, wherein the click signal is generated by a separate click mechanism.

3. The method according to claim 1, wherein the click signal is generated by a click mechanism (2) which is arranged on a medical instrument (1).

4. The method according to any one of claims 1 to 3, wherein two or more click signals are generated which differ in particular in tone and/or successive click signals are generated in different rhythms.

5. The method according to any one of claims 1 to 4, wherein different image outputs are provided within the scope of navigation, and wherein receiving the click signal changes the image output.

6. The method according to claim 5, wherein switching the image output over, forwards or backwards is triggered by the click signal, in order to change the different image outputs.

7. The method according to any one of claims 1 to 4, wherein one or more of the following actions are triggered by the click signal:
- importing image data sets into the navigation system;
- beginning a registration or re-registration for parts of the patient's body or instruments; and
- launching a treatment-assisting apparatus.

8. A device for controlling a medical navigation system (4), comprising a medical navigation and/or image-assisting system (4) and a control for the system in response to an input, **characterised in that** the control includes a click device (2) and a receiving device (3) for a click signal, assigned to the navigation system.

9. The device according to claim 8, **characterised in that** it includes a separate click mechanism.

10. The device according to claim 8, **characterised in that** it includes a click mechanism (2) which is arranged on a medical instrument (1), in particular arranged such that it can be removed and/or replaced.

11. The device according to any one of claims 8 to 10, **characterised in that** the control is integrated in the navigation system (4).

12. The device according to any one of claims 8 to 11, **characterised in that** the click mechanism includes a deformable planar structure, preferably made of metal, which experiences an abrupt change in shape in a portion, in particular a pre-shaped portion, by mechanical deformation.

13. The device according to any one of claims 8 to 12, comprising a click mechanism which can generate two or more click signals which differ in tone, in particular in pitch, in particular by changing the shape of different portions or of the same portion in different directions.

14. The device according to any one of claims 8 to 13, **characterised in that** it comprises two or more click mechanisms in order to generate different click signals.

15. The device according to any one of claims 8 to 14, **characterised in that** the navigation system includes an image output unit (5), and the control controls the display on the image output unit (5), specifically in order to change the image output, in particular in order to switch the image output forwards or backwards in an image output sequence of a software workflow.

## Revendications

1. Procédé de commande d'un système de navigation (4) médical, assisté par ordinateur, par lequel est garantie une assistance pour une partie déterminée d'un traitement, **caractérisé en ce qu'**un signal de clic est produit et reçu par le système de navigation, après quoi le système de navigation (4) active l'assistance au traitement.

2. Procédé selon la revendication 1 dans lequel le signal de clic est produit par un mécanisme de clic séparé.

3. Procédé selon la revendication 1 dans lequel le signal de clic est produit avec un mécanisme de clic qui est disposé sur un instrument médical (1).

4. Procédé selon l'une des revendications 1 à 3, dans lequel sont produits deux signaux de clic ou plus, en particulier des signaux de clic de son différent et/ou se succédant à un rythme différent.

5. Procédé selon l'une des revendications 1 à 4, dans lequel différentes sorties images sont produites dans le cadre de la navigation, et dans lequel la réception du signal de clic provoque le changement de la sortie image.

6. Procédé selon la revendication 5, dans lequel le signal de clic déclenche la commutation ou l'avance ou le retour de la sortie image, afin de provoquer différents changements de sorties images.

7. Procédé selon l'une des revendications 1 à 4, dans lequel une ou plusieurs des actions suivantes sont déclenchées par le signal de clic :
- l'introduction d'ensembles de données d'image dans le système de navigation ;
- le début d'un enregistrement ou d'un enregistrement complémentaire pour des parties corporelles d'un patient ou des instruments ; et
- le démarrage ou l'approche d'un appareil d'assistance au traitement.

8. Dispositif pour la commande d'un système de navigation (4) médical avec un système médical de navigation et/ou d'assistance par imagerie et avec une commande pour le système en réaction à une saisie, **caractérisé en ce que** la commande comprend un dispositif de clic (2) et un dispositif de réception (3), associé au système de navigation, pour un signal de clic.

9. Dispositif selon la revendication 8, **caractérisé en ce qu'**il comprend un mécanisme de clic séparé.

10. Dispositif selon la revendication 8, **caractérisé en ce qu'**il comprend un mécanisme de clic (2) qui est disposé sur un instrument médical (1), en particulier de manière amovible et/ou échangeable.

11. Dispositif selon l'une des revendications 8 à 10, **caractérisé en ce que** la commande est intégrée dans le système de navigation (4).

12. Dispositif selon l'une des revendications 8 à 12, **caractérisé en ce que** le mécanisme de clic comprend une structure plate déformable, de préférence en métal, qui subit un changement de forme brusque d'un segment, en particulier préformé, par une déformation mécanique.

13. Dispositif selon l'une des revendications 8 à 12, avec un mécanisme de clic qui peut produire deux signaux de clic ou plus, différents par leur son, en particulier la hauteur du son, notamment par déformation de différents segments ou du même segment dans différentes directions.

14. Dispositif selon l'une des revendications 8 à 13, **caractérisé en ce qu'**il comporte deux mécanismes de clic ou plus pour produire différents signaux de clic.

15. Dispositif selon l'une des revendications 8 à 14, **caractérisé en ce que** le système de navigation comprend une unité de sortie image (5) et la commande commande la représentation sur l'unité de sortie image (5), en particulier pour provoquer le changement de la sortie image, notamment pour l'avance ou le retour de la sortie image dans une succession de sorties images d'une séquence de logiciel.
